# EUROPÄISCHE PATENTANMELDUNG

(11) **EP 1 169 999 A2**
(43) Veröffentlichungstag der Anmeldung: **09.01.2002**
(21) Anmeldenummer: 01810406.7
(22) Anmeldetag: 25.04.2001
(51) Int. Cl.: A61K 7/06

(54) **Haarbehandlungsmittel in Aerosolform zur Unterdrückung von unangenehmen Düften auf menschlichem oder tierischem Haar**

(30) Priorität: 06.07.2000 CH 133500
(71) Anmelder: Mibelle AG Cosmetics, 5033 Buchs (CH)
(72) Erfinder: Karlen, Thomas, Dr., 4056 Basel (CH)
(74) Vertreter: Rottmann, Maximilian R.

(57) **Zusammenfassung**

Das Haarbehandlungsmittel in Form eines Aerosolsprays zur Unterdrückung von unangenehmen Düften auf menschlichem oder tierischem Haar ist gekennzeichnet durch einen Gehalt an: (A) mindestens 0,05 Gewichtsprozent eines geruchsneutralisierenden Stoffes oder Stoffgemisches; und (B) mindestens 5 Gewichtsprozent eines Treibgases oder Treibgasgemisches in verflüssigter Form. Die Anwendung dieses Mittels in Spray-form auf übel riechendes Haar vermindert den unangenehmen Duft auf dem Haar, ohne dass beim Menschen das Haar belastet wird oder die bestehende Frisur beeinrächtigt wird und ohne dass bei Tieren der Griff und der Glanz des Felles beeinflusst wird.

## Beschreibung

Die Erfindung betrifft:
- ein Haarbehandlungsmittel, wie es im Anspruch 1 umschrieben ist; und
- die gewerbliche Verwendung eines Haarbehandlungsmittels, wie sie im Anspruch 11 umschrieben ist.
Bevorzugte Ausführungsformen sind in den abhängigen Ansprüchen umschrieben.

Haarbehandlungsmittel dienen dazu, das Haar in Form zu bringen (Styling, Dauerwelle), das Haar zu färben (Färbemittel) oder das Haar zu pflegen (Shampoos, Conditioner).

Um dem Haar einen angenehmen Geruch zu verleihen, enthalten diese Produkte meistens ein Parfüm, welches den Zweck hat, unangenehme Düfte, wie Essensdüfte oder Zigarettenduft, zu überdecken und dem Haar eine bestimmte Geruchsnote zu verleihen. Der Nachteil dieses Vorgehens besteht darin, dass der natürliche Duft des sauberen Haares überdeckt wird durch den künstlichen Duft des Parfüms.

Es existieren auf dem Markt bereits Produkte in Sprayform, welche zum Zwecke der Verminderung von unangenehmen Düften auf dem Haar Parfümöle zur Überdeckung dieser Düfte enthalten. Auch hier wird der unangenehme Duft lediglich durch einen stärkeren "wohlriechenden" Duft maskiert.

Es sind überdies Mittel im Haarfärbe- und Dauerwellbereich bekannt, welche geruchsneutralisierende Rohstoffe zum Zweck der Verminderung von unangenehmen Gerüchen während der Applikation der Mittel enthalten (EP-A1-0 723 772, EP-A1-0 713 695, EP-A-0 354 835, DE-A-34 33 648, JP-A-48 085 745).

Breit eingesetzt werden geruchsneutralisierende Substanzen im Bereich der Körperdeodorants, der Lufterfrischer sowie in Haushaltsprodukten, wie beispielsweise Kleiderauffrischer, Teppichreiniger oder Haushaltsseifen.

Aufgabe der vorliegenden Erfindung ist die Schaffung eines Haarbehandlungsmittels, welches übel riechende Düfte auf menschlichem oder tierischem Haar neutralisiert ohne den Eigengeruch des Haares zu beeinträchtigen und ohne es zu belasten.

Diese Aufgabe wird durch das im Kennzeichen des Anspruchs 1 umschriebene Haarbehandlungsmittel gelöst. Es liegt in Form eines Aerosolsprays vor.

Als "geruchsneutralisierend" wird die Eigenschaft des Rohstoffs der Komponente (A) verstanden, den Dampfdruck von übel riechenden Düften herabzusetzen, indem er die übel riechenden Duftkomponenten entweder neutralisiert oder adsorbiert.

Beispiele für geeignete Rohstoffe der Komponente (A) sind die unter der Sammelbezeichnung "Odour Neutralizer" zusammengefassten Öle der Firma Haarmann & Reimer GmbH, Postfach 1253, D-37601 Holzminden, wie beispielsweise:
Odour Neutralizer D61012 /780 134
Odour Neutralizer D61012A / 785 553
Odour Neutralizer D61012B /785 554
Odour Neutralizer D61012C /785 555.

Weitere Beispiele für geeignete Rohstoffe der Komponente (A) sind die von der Firma Th. Goldschmidt AG, Essen/Deutschland, unter der Bezeichnung "TEGO® Deo" angebotenen Rohstoffe mit einem Gehalt an Zinkricinoleat (INCI-Bezeichnung: Zinc Ricinoleate), beispielsweise TEGO® Deo CW 90 oder TEGO® Deo HY 77.

Vorzugsweise sind die Rohstoffe der Komponente (A) im Treibgas oder Treibgasgemisch der Komponente (B) löslich.

Zweckmässigerweise enthält das erfindungsgemässe Haarbehandlungsmittel 5 bis 95 Gewichtsprozent, vorzugsweise 10 bis 85 Gewichtsprozent, des Treibgases der Komponente (B). Es wird in einen Druckbehälter abgefüllt. Als Treibgase sind beispielsweise niedere Alkane, wie n-Butan, Isobutan und Propan, oder Dimethylether oder Gemische dieser Stoffe geeignet.

Das erfindungsgemässe Haarbehandlungsmittel wird vorzugsweise in einem alkoholischen oder wässrig-alkoholischen Milieu konfektioniert. Als Alkohole können insbesondere die für kosmetische Zwecke üblicherweise verwendeten niederen Alkohole mit 1 bis 4 Kohlenstoffatomen, wie beispielsweise Ethanol oder Isopropanol, eingesetzt werden.

Des weiteren können Lösungsmittel oder Lösungsmittel-Gemische mit einem Siedepunkt unter 400 °C in einer Menge von 0,1 bis 90 Gewichtsprozent, vorzugsweise von 1 bis 50 Gewichtsprozent, eingesetzt werden. Besonders geeignet sind unverzweigte und verzweigte Kohlenwasserstoffe, wie Pentan, Hexan, Isopentan, sowie cyclische Kohlenwasserstoffe, wie Cyclopentan und Cyclohexan. Weitere, besonders bevorzugte wasserlösliche Lösungsmittel sind Glycerin und Propylenglykol in einer Menge von bis zu 30 Gewichtsprozent.

Ist Wasser vorhanden, so kann das erfindungsgemässe Haarbehandlungsmittel in einem pH-Bereich von 2,0 bis 9,5 vorliegen. Besonders bevorzugt ist der pH-Bereich von 2,5 bis 8. Liegt das erfindungsgemässe Haarbehandlungsmittel im sauren Bereich vor, so kann es organische oder anorganische Säuren enthalten, wie beispielsweise Ameisensäure, Weinsäure, Äpfelsäure, Maleinsäure, Fumarsäure, Pyrrolidoncarbonsäure, Citronensäure, Schwefelsäure, Essigsäure, Salzsäure und/oder Phosphorsäure.

Das erfindungsgemässe Haarbehandlungsmittel kann zusätzlich Rohstoffe enthalten, welche üblicherweise in Aerosolsprays für die Haarkosmetik verwendet werden. Beispiele sind Kämmbarkeitsverbesserer, insbesondere kationische Tenside, kationische Polymere oder kationische Silikonverbindungen. Des weiteren können filmbildende haarfestigende kationische, anionische, amphotere oder nichtionische Polymere mitverwendet werden. Ebenso können nichtionische, amphotere oder anionische Tenside oder Emulgatoren eingesetzt werden.

Unter filmbildenden, haarfestigenden Polymeren sollen solche Polymere verstanden werden, welche allein in 0,1- bis 5%iger wässriger, alkoholischer oder wässrig-alkoholischer Lösung angewendet, in der Lage sind, auf dem Haar einen Polymerfilm abzuscheiden und auf diese Weise das Haar zu festigen.

Selbstverständlich können die erfindungsgemässen Haarbehandlungsmittel auch weitere übliche kosmetische Zusätze enthalten, beispielsweise:
- nichtfestigende nichtionische Polymere, wie Polyethylenglykole oder Copolymere aus Ethylenglykol und Propylenglykol; nichtfestigende anionische Polymere; und nichtfestigende natürliche Polymere sowie deren Kombination, in einer Menge von vorzugsweise 0,01 bis 50 Gewichtsprozent;
- Parfümöle, in einer Menge von vorzugsweise 0,01 bis 5 Gewichtsprozent;
- Trübungsmittel, wie Ethylenglykoldistearat, in einer Menge von vorzugsweise 0,01 bis 5 Gewichtsprozent;
- Netzmittel oder Emulgatoren aus den Klassen der anionischen, kationischen, amphoteren oder nichtionogenen oberflächenaktiven Substanzen, wie Fettalkoholsulfate, ethoxylierte Fettalkohole, Fettsäurealkanolamide, wie die Ester der hydrierten Ricinusölfettsäuren, in einer Menge von vorzugsweise 0,1 bis 30 Gewichtsprozent; ferner
- Feuchthaltemittel, Farbstoffe, Lichtschutzmittel, Antioxidantien, Glanzgeber und Konservierungsstoffe, in einer Menge von vorzugsweise 0,01 bis 10 Gewichtsprozent.

Als weitere Zusätze sind geeignete Silikonpolymere einsetzbar, beispielsweise:
- Polydimethylsiloxan (INCI-Bezeichnung: Dimethicon);
- α-Hydro-ω-hydroxypolyoxydimethylsilylen (INCI-Bezeichnung: Dimethiconol);
- cyclisches Dimethylpolysiloxan (INCI-Bezeichnung: Cyclomethicon);
- Trimethyl(octadecyloxy)silan (INCI-Bezeichnung: Stearoxytrimethylsilan);
- Dimethylsiloxan-Glykol-Copolymer (INCI-Bezeichnung: Dimethicon Copolyol);
- Dimethylsiloxan-Aminoalkylsiloxan-Copolymer mit Hydroxyendgruppen (INCI-Bezeichnung: Amodimethicon)
- Monomethylpolysiloxan mit Laurylseitenketten und Polyoxyethylen- und/oder Polyoxypropylenendketten (INCI-Bezeichnung: Laurylmethicon Copolyol);
- Dimethylsiloxan-Glykol-Copolymer-Acetat (INCI-Bezeichnung: Dimethicone Copolyol Acetate);
- Dimethylsiloxan-Aminoalkylsiloxan-Copolymer mit Trimethylsilylendgruppen (INCI-Bezeichnung: Trimethylsilylamodimethicon).

Bevorzugte Silikonpolymere sind:
- Dimethicone, welche beispielsweise von der Firma Wacker, München/Deutschland unter der Handelsbezeichnung SILOXANE F-221 oder von der Firma Dow Corning Europe, Brüssel/Belgien unter der Handelsbezeichnung DOW CORNING FLUID 200/0,65 cs vertrieben werden;
- Cyclomethicone, welche beispielsweise von der Firma Dow Corning Europe, Brüssel/Belgien unter der Handelsbezeichnung DOW CORNING 244 FLUID oder von der Firma Th. Goldschmidt, Essen/Deutschland unter der Handelsbezeichnung ABIL® K4 vertrieben werden;
- Dimethiconole, welche beispielsweise von der Firma Wacker/Deutschland unter der Handelsbezeichnung SILICONE FLUID F-212 oder von der Firma Universal Preserv-A-Chem, Inc., 33 Truman Drive South, Edison NJ, USA, unter der Handelsbezeichnung UNISIL® SF-R vertrieben werden.

Die vorstehend in Klammern angegebenen Bezeichnungen entsprechen der INCI-Nomenklatur (International Cosmetics Ingredients), wie sie zur Kennzeichnung kosmetischer Wirk- und Hilfsstoffe bestimmt ist.

Auch Gemische von Silikonpolymeren sind geeignet, wie z.B. ein Gemisch aus Dimethicon und Dimethiconol, welches beispielsweise von der Firma Dow Corning Europe/Belgien unter der Handelsbezeichnung DOW CORNING 1403 FLUID vertrieben wird.

Unter Haarbehandlung soll sowohl die Behandlung des menschlichen Kopfhaares, vor allem zum Zwecke der Erzeugung einer Frisur oder zur Pflege der Haare, als auch die Behandlung des Felles von Tieren verstanden werden.

Bei der Anwendung des erfindungsgemässen Haarbehandlungsmittels durch Besprühen von menschlichem Haar, welches durch Einflüsse übel riechender Düfte einen unangenehmen Duft erhalten hat, wird innerhalb von 5 Minuten eine weitgehende Reduktion der übel riechenden Düfte erreicht, ohne dass das Haar belastet oder eine bestehende Frisur beeinträchtigt wird.

Bei der Anwendung des erfindungsgemässen Haarbehandlungsmittels auf tierischem Haar wird der Griff und der Glanz des Felles nicht negativ beeinflusst.

Die nachstehenden Beispiele sollen den Erfindungsgegenstand näher erläutern. Alle Angaben sind in Gewichtsprozent.

### Beispiel 1

Mittel zur Verminderung unangenehmer Gerüche auf dem Haar, wasserfrei

| | |
|---|---|
| Alkohol absolut | 10,00% |
| Odour Neutralizer D61012B | 1,00 % |
| Propan/Butan 4,1 Bar | 89,00 % |

Die Anwendung dieses Mittels in Sprayform auf übel riechendes Haar vermindert den unangenehmen Duft auf dem Haar, ohne dass das Haar belastet wird oder die bestehende Frisur beeinträchtigt wird.

### Beispiel 2

Mittel zur Verminderung unangenehmer Gerüche auf dem Haar, mit Wasser

| | |
|---|---|
| Alkohol denaturiert | 20,00 % |
| Wasser | 20,00 % |
| Odour Neutralizer D61012B | 1,00% |
| Dimethylether | 59,00 % |

Die Anwendung dieses Mittels in Sprayform auf übel riechendes Haar vermindert den unangenehmen Duft auf dem Haar, ohne dass das Haar belastet wird oder die bestehende Frisur beeinträchtigt wird.

### Beispiel 3

Mittel zur Verminderung unangenehmer Gerüche auf dem Haar, mit Glanzgeber

| | |
|---|---|
| Alkohol denaturiert | 20,00 % |
| Odour Neutralizer D61012B | 1,00 % |
| Dimethicone | 2,00 % |
| Propan/Butan 4,1 Bar | 77,00 % |

Die Anwendung dieses Mittels in Sprayform auf übel riechendes Haar vermindert den unangenehmen Duft auf dem Haar, ohne dass das Haar belastet wird oder die bestehende Frisur beeinträchtigt wird.

### Beispiel 4

Mittel zur Verminderung unangenehmer Gerüche auf dem Haar, mit Pflegewirkstoff

| | |
|---|---|
| Alkohol denaturiert | 20,00 % |
| Wasser | 20,00 % |
| Odour Neutralizer D61012B | 1,00 % |
| Panthenol | 0,30 % |
| Dimethylether | 59,00 % |

Die Anwendung dieses Mittels in Sprayform auf übel riechendes Haar vermindert den unangenehmen Duft auf dem Haar, ohne dass das Haar belastet wird oder die bestehende Frisur beeinträchtigt wird.

### Beispiel 5

Mittel zur Verminderung unangenehmer Gerüche auf dem Haar, mit Volumengeber

| | |
|---|---|
| Alkohol denaturiert | 20,00 % |
| Wasser | 20,00 % |
| Odour Neutralizer D61012B | 1,00% |
| PVP K 80 | 0,20 % |
| Dimethylether | 59,00 % |

Die Anwendung dieses Mittels in Sprayform auf übel riechendes Haar vermindert den unangenehmen Duft auf dem Haar, ohne dass das Haar belastet wird oder die bestehende Frisur beeinträchtigt wird.

### Wirksamkeitsprüfung

Die Formulierung von Beispiel 1 wurde folgendermassen getestet:

20 Probanden (unbehandelte Gruppe) wurden während 10 Minuten in einem Raum starker Belastung von Zigarettenrauch ausgesetzt. 5 Minuten nach Verlassen des Raumes wurde der Haarduft der Probanden von 5 Testern beurteilt. 15 Minuten nach Verlassen des Raumes wurde nochmals beurteilt. Der Duft wurde ebenfalls von den Probanden selbst beurteilt.

Eine zweite Gruppe (behandelte Gruppe) von 20 Probanden wurde während 10 Minuten in einem Raum starker Belastung von Zigarettenrauch ausgesetzt. Unmittelbar nach Verlassen des Raumes applizierten die Probanden das erfindungsgemässen Haarbehandlungsmittel gemäss Beispiel 1. Der Haarduft der Probanden wurde danach ebenfalls sowohl durch die Probanden selbst als auch durch die 5 Tester nach 5 sowie nach 15 Minuten kontrolliert.

Die Durchschnittswerte der Resultate der beiden Probandengruppen sind nachstehend in Tabellenform zusammengestellt.

| | Duft nach 5 Minuten | Duft nach 15 Minuten |
|---|---|---|
| Selbstkontrolle Gruppe unbehandelt | 1,5 | 2,1 |
| Selbstkontrolle Gruppe behandelt | 3,2 | 3,5 |
| Kontrolle Tester Gruppe unbehandelt | 1,2 | 1,9 |
| Kontrolle Tester Gruppe behandelt | 3,1 | 3,6 |

Skala Dufteindruck:
1 = sehr unangenehm
2 = ziemlich unangenehm
3 = leicht unangenehm
4 = angenehm

Die Resultate zeigen deutlich den positiven Einfluss des erfindungsgemässen Haarbehandlungsmittels hinsichtlich Geruchsbeeinflussung auf dem Haar durch Selbst- sowie durch Fremdeinschätzung.

## Patentansprüche

1. Haarbehandlungsmittel in Form eines Aerosolsprays zur Unterdrückung von unangenehmen Düften auf menschlichem oder tierischem Haar, **gekennzeichnet durch** einen Gehalt an:
(A) mindestens 0,05 Gewichtsprozent eines geruchsneutralisierenden Stoffes oder Stoffgemisches; und
(B) mindestens 5 Gewichtsprozent eines Treibgases oder Treibgasgemisches in verflüssigter Form.

2. Haarbehandlungsmittel nach Anspruch 1, **dadurch gekennzeichnet, dass** es 0,1 bis 3 Gewichtsprozent des geruchsneutralisierenden Stoffes oder Stoffgemisches enthält.

3. Haarbehandlungsmittel nach Anspruch 1 oder 2, **dadurch gekennzeichnet, dass** es 5 bis 95 Gewichtsprozent des Treibgases oder Treibgasgemisches enthält.

4. Haarbehandlungsmittel nach Anspruch 3, **dadurch gekennzeichnet, dass** es 10 bis 85 Gewichtsprozent des Treibgases oder Treibgasgemisches enthält.

5. Haarbehandlungsmittel nach einem der Ansprüche 1 bis 4, **dadurch gekennzeichnet, dass** es als Treibgas n-Butan, Isobutan, Propan, Dimethylether oder Gemische dieser Stoffe enthält.

6. Haarbehandlungsmittel nach einem der Ansprüche 1 bis 5, **dadurch gekennzeichnet, dass** es in einem alkoholischen oder wässrig-alkoholischen Milieu konfektioniert ist.

7. Haarbehandlungsmittel nach einem der Ansprüche 1 bis 6, **dadurch gekennzeichnet, dass** es ausserdem ein Lösungsmittel oder Lösungsmittelgemisch mit einem Siedepunkt unter 400 °C enthält.

8. Haarbehandlungsmittel nach Anspruch 7, **dadurch gekennzeichnet, dass** es 0,1 bis 90 Gewichtsprozent des Lösungsmittels enthält.

9. Haarbehandlungsmittel nach Anspruch 8, **dadurch gekennzeichnet, dass** es 1 bis 50 Gewichtsprozent des Lösungsmittels enthält.

10. Haarbehandlungsmittel nach einem der Ansprüche 1 bis 7, **dadurch gekennzeichnet, dass** es ausserdem eine oder mehrere Substanzen oder Substanzgemische der folgenden Stoffklassen enthält:
- Kämmbarkeitsverbesserer;
- filmbildende haarfestigende kationische, anionische, amphotere oder nichtionische Polymere;
- nichtionische, amphotere oder anionische Tenside oder Emulgatoren;
- nichtfestigende nichtionische, anionische oder natürliche Polymere;
- Parfümöle;
- Trübungsmittel;
- Netzmittel;
- Emulgatoren;
- Feuchthaltemittel;
- Farbstoffe;
- Lichtschutzmittel;
- Antioxidantien;
- Glanzgeber;
- Konservierungsstoffe;
- Silikonpolymere.

11. Gewerbliche Verwendung eines Haarbehandlungsmittel nach einem der Ansprüche 1 bis 10 zur Duftneutralisierung auf menschlichem oder tierischem Haar.
